(19) Europäisches Patentamt
European Patent Office
Office européen des brevets

(11) **EP 2 568 404 A1**

(12) **EUROPEAN PATENT APPLICATION**

(43) Date of publication:
**13.03.2013 Bulletin 2013/11**

(51) Int Cl.:
***G06F 19/00*** *(2011.01)*

(21) Application number: **12183425.3**

(22) Date of filing: **07.09.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.09.2011 US 201161532349 P**
**30.08.2012 US 201213599262**

(71) Applicant: **The Charlotte-Mecklenburg Hospital Authority**
**Charlotte, NC 28203 (US)**

(72) Inventor: **Kline, Jeffrey A.**
**Charlotte, NC North Carolina 28216 (US)**

(74) Representative: **Patentanwälte**
**Ruff, Wilhelm, Beier, Dauster & Partner**
**Kronenstrasse 30**
**70174 Stuttgart (DE)**

(54) **A computer-based device and model for predicting probability of death from thrombosis**

(57) A computer-based device and predictive model executable by computer software for use with the device to predict or estimate a percentage probability of death from thrombosis in a patient with active cancer.

*FIG. 3*

**Description**

CROSS-REFERNCE TO RELATED APPLICATIONS

This application claims priority from United States Provisional Patent Application No. 61/532,349, filed on September 8, 2011, incorporated herein by reference in its entirety.

FIELD OF THE INVENTION

The present invention relates to a computer-based device and model for predicting or estimating probability of death from thrombosis.

BACKGROUND OF THE INVENTION

Thrombosis generally refers to formation of a blood clot inside a blood vessel such as a vein or artery. Among the common types of thrombosis are venous thrombosis and arterial thrombosis. One complication that can result from thrombosis is pulmonary embolism (PE). Pulmonary embolism is basically a blockage of the main artery of the lung or one of its branches by a substance such as a blood clot that has travelled from elsewhere in the body through the blood stream. The risk of pulmonary embolism is increased in various medical situations and with certain medical conditions.

Clinical guidelines recommend risk stratification of patients with acute pulmonary embolism. Risk stratification of acute pulmonary embolism generally refers to the use of objective criteria to predict the probability of clinical deterioration during a short term follow-up period after diagnosis. Active cancer increases risk of PE and worsens prognosis but also causes incidental PE discovered during cancer staging.

The majority of published literature focuses on categorizing patients as low, moderate, or high risk of death, respiratory failure, or circulatory failure during the inpatient period or up to 30 days. Known methods of risk stratification include the use of echocardiography, biomarkers, scoring systems, CT scan results, and use of comborbid conditions. Increased risk has been suggested to be important in decisions regarding need for admission, or need for treatment beyond standard anticoagulation. Patients with active malignancy represent a subgroup at particularly high risk of both developing PE and having an increased risk of death after diagnosis of PE compared with patients who have no history of cancer or a past medical history of cancer in remission at the time of PE diagnosis. At present, there is no known device for estimating the quantitative short-term prognosis of a patient with active malignancy and PE. No quantitative decision instrument or device has been derived specifically for patients with active cancer and PE. Thus, the present invention is directed to a computer-based device having a multivariate, quantitative computer-implemented modeling equation used for the purpose of assessing the probability of death within 30 days of patients with active cancer and PE.

SUMMARY OF THE INVENTION

The present invention is directed to a computer-based device and predictive model to estimate a quantitative short-term prognosis of a patient with active cancer and thrombosis. The present invention is directed to a computer-based device and predictive model to estimate a quantitative short-term prognosis of a patient with active cancer and pulmonary embolism. The present invention is further directed to a computer-based device and predictive model executable by computer software for use with the device to predict a percentage probability of death from pulmonary embolism in a patient with active cancer.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

BRIEF DESCRIPTION OF THE DRAWINGS

The present invention will become more fully understood from the detailed description and the accompanying drawings, which are not necessarily to scale, wherein:

Figure 1 is an illustrative computer screen shot from a computer-based device showing the output resulting from the predictive model of the present invention.

Figure 2 is a receiver operating characteristic curve using a percentage probability estimate of mortality from a logistic regression equation as the diagnostic test, where the outcome was 30 day mortality in the validation dataset.

Figure 3 is a dot plot of the estimated probability of death from a logistic regression equation (Y axis), stratified according to survival status at 30 days (X axis).

DETAILED DESCRIPTION OF THE INVENTION

The following detailed description of the embodiment(s) is merely exemplary in nature and is in no way intended to limit the invention, its application, or uses.

The computer-based device and predictive model of the present invention is used to estimate a quantitative short-term prognosis of a patient with active cancer and thrombosis. The computer-based device and predictive model of the present invention is also used to estimate a quantitative short-term prognosis of a patient with active cancer and pulmonary embolism.

The computer-based device comprises a predictive model executable by computer software for estimating a percentage probability of death from pulmonary embolism (PE) in a patient with active cancer. The computer-based device typically comprises a processor, a data input device (e.g., keyboard), a data output device (e.g. computer monitor or display computer screen), a disk drive, a computer software application, computer memory, and data storage modules. The computer memory comprises instructions executable by the processor to run the predictive model of the present invention. The computer device may be communicatively connected to a computer network.

The predictive model of the present invention comprises a multivariate equation. As a feature of the model, the multivariate equation is a logistic regression equation. As another feature of the model, the equation typically has eight variables or factors. The model has surprisingly been found to have good prognostic accuracy for 30 day mortality especially at low and high estimates. The model is helpful for cancer patients with PE discovered during disease staging and near the end of life. Additionally, the model has good overall diagnostic accuracy for prediction of death in patients with malignancy and suspected PE but for whom no PE was found despite standard of care evaluation for PE.

Equation (1) illustrates a multivariate equation for use in the model of the present invention in a form of a logistic regression equation. The logistic function may be plotted with z on the horizontal axis and f(z) on the vertical axis.

$$f(z) = 1/(1+e^{-z}) \qquad \qquad \text{Equation (1)}$$

wherein

$f(z)$ = output (i.e. probability of particular outcome)
$z$ = input = $\beta_0 + \beta_1 x_1 + \beta_2 x_2 + \beta_3 x_3 + \beta a x4... + \beta_k x_k$
$x_1, x_2, x_3, ... x_i$ = factors
$k = 1,2,3,...i$
$\beta_0$ = intercept
$\beta_1, \beta_2, \beta_3, ... \beta_k$ = regression coefficients

Equation (2) is a derived logistic regression equation in the form of equation (1) having the following unique intercept, regression coefficients and factors for assessing the probability of death from pulmonary embolism (PE) in a patient with active cancer within thirty days.

$$P(z) = [1 - [1/(1+e^{z})]] * 100 \qquad \qquad \text{Equation (2)}$$

wherein

$P(z)$ = percentage probability of death within thirty days
$z = \beta_0 + \beta_1 x_1 + \beta_2 x_2 + \beta_3 x_3 + \beta_4 x_4 + \beta_5 x_5 + \beta_6 x_6 + \beta_7 x_7 + \beta_8 x_8$
$\beta_0$ = intercept
$\beta_1$ = patient body weight
$\beta_2$ = heart rate
$\beta_3$ = respiratory rate
$\beta_4$ = pulse oximetry ($SaO_2$)
$\beta_5$ = altered mental state
$\beta_6$ = respiratory distress
$\beta_7$ = "do not resuscitate" status
$\beta_8$ = unilateral limb swelling

wherein

$x_1$ = body weight of patient in pounds

$x_2$ = 1 if heart rate of patient > 99 beats per minute

but $x_2$ = 0 if heart rate <100 beats per minute

$x_3$ = respiratory rate of patient in breaths per minute

$x_4$ = pulse oximetry value in % obtained from the patient breathing room air

$x_5$ = 1 if patient has altered mental state but $x_5$ = 0 if patient does not have altered mental state

$x_6$ = 1 if patient has respiratory distress but $x_6$ = 0 if patient does not have respiratory distress

$x_7$ = 1 if patient has "do not resuscitate" status but $x_7$ = 0 if patient does not have "do not resuscitate status"

$x_8$ = 1 if patient has unilateral limb swelling but $x_8$ = 0 but if patient does not have unilateral limb swelling

As indicated above in Equation (2), the multivariate equation has eight predictor factors $x_1$ to $x_8$. The factors are body weight, heart rate, respiratory rate, pulse oximetry, respiratory distress, altered mental status, do not resuscitate status and unilateral limb swelling.

The beta coefficients of equation (2) preferably have the following values:

$\beta_0$ = 3.72

$\beta_1$ is in a range of -0.02 to 0

$\beta_2$ is in a range of 0.35 to 1.83

$\beta_3$ is in a range of -0.02 to 0.11

$\beta_4$ is in a range of -0.13 to 0

$\beta_5$ is in a range of 0.05 to 2.58

$\beta_6$ is in a range of -0.12 to 1.48

$\beta_7$ is in a range of 0.29 to 2.66

$\beta_8$ is in a range of -0.16 to 1.53

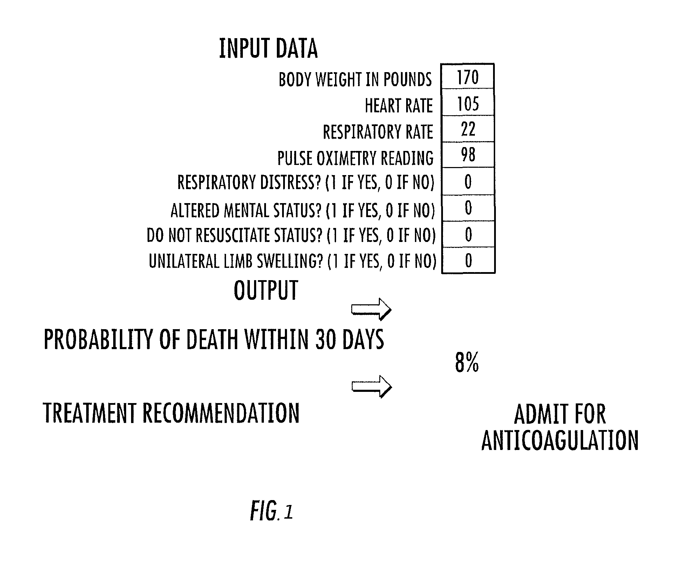

In accordance with the present invention, a user enters the values for the factors in the computer-based device using a computer user interface or application, such as an "Applet" programmed in Javascript, or HTML, or a macro programmed in Visual Basic for the user to input the variables or input data into the device and computer code to execute the equation and present the percentage probability of death within 30 days. In addition to the values for the factors, the user enters any other needed information. The input data once entered is processed according to the computer-implemented model to generate the output data. The output data is displayed on a computer screen or other output display device as illustrated in Figure 1, and the output data includes, but is not limited to, a percentage probability of death within the next 30 days and a recommended course of action based upon the percentage probability of death. The course of action may include, but is not limited to, treatment options and physical location of the patient, either in the hospital or home setting. It is desirable for the variables to be obtained at the bedside of the patient and for the device to be used in association with a medical history review and physical examination of a patient.

A percentage of 5% or less corresponds to a near zero chance of death within 30 days and therefore the device output is typically a recommendation of home treatment. A percentage of >50% corresponds to a high probability of death with standard treatment, suggestive of need for advanced treatment options, including fibrinolysis, catheter embolectomy, or de-escalation of medical care and transition to palliation. At a percentage of 6 to 49% the device output is typically a recommendation of hospital admission with standard anticoagulation. The device is optionally used in conjunction with an anticoagulant treatment to allow early discharge of patients with cancer and PE.

It is within the scope of the present invention that the variables are modifiable slightly either by substitution or addition. For example, unilateral limb swelling can be substituted for one of the following: prior PE, systolic blood pressure, immobility, and alternative diagnosis to obtain performance almost as good. Similarly, the variable of breathing room air could substitute for respiratory distress. Likewise, the intercept and regression coefficients are able to be changed slightly and the device performance nearly maintained. The device is able to be augmented to estimate other outcomes relevant to treatment options including probability of bleeding.

It is within the scope of the present invention that variables are able to be added or substituted, coefficients modified, or the contents of the equation used in different prediction models, including classification trees, nomograms, Bayesian network, artificial intelligence, nearest neighbor, or other nonlinear techniques.

The results achieved with the derived multivariate model and device of the present invention are surprising, especially the accuracy of predicting near zero probability of death in one-third of the at risk population. There is a need for the device and model of the present invention which stems from the need for a validated clinical pathway to manage cancer patients who undergo staging imaging that includes the contrast-enhanced imaging pulmonary vasculature, which demonstrates an incidentally discovered PE. This occurs in approximately 1.5-2.5% of scans. Not all incidental clots are benign especially in the case of larger clots. The device and model of the present invention are suitable for use in a clinical trial of outpatient management for patients with active cancer and PE, including those with incidentally discovered PE, and with a mortality estimate of 5% or less. Additionally, for selected patients with advanced cancer and PE, an accurate quantitative prediction device and model may also provide better congruence between what families and patients want and the care that providers give them near the end of life decisions.

EXAMPLE

The model of the present invention was derived using a subset of 408 patients with active cancer and PE who were enrolled in a multicenter registry known as Emergency Medicine Pulmonary Embolism in the Real World Registry (EMPORER). The dataset was collected in 22 community and academic emergency departments in the United States over a three year period.

Twenty-five (25) potential candidate predictor variables were selected; combined use of univariate (P<0.1) and classification and regression tree analysis selected 8 final predictors which were entered into multivariate logistic regression, solved for P to predict 30 day mortality. The resultant model cross validated in a composite sample of 149 patients with active cancer and PE. The model (body mass, heart rate >100, respiratory rate, SaO2%, respiratory distress, altered mental status, do not resuscitate status, unilateral limb swelling) had an area under the receiver operating characteristic curve of 0.83 in the derivation set and 0.85 (95% CI 0.73-0.95) in the validation sample. In the validation sample, no patient with probability estimate ≤5% died (0/42, 95% CI 0 to 8.4%) whereas 8/11 (73%, 95% CI 39 to 94%) with an estimate >50% died within 30 days.

Participating sites in EMPORER prospectively collected 264 data elements on 1,880 patients diagnosed with PE in the emergency department, including 30 day outcome.

The device of the present invention collected detail about risk factors for PE as well as data to predict adverse outcomes. The device collected demographic data, vital signs, comorbidities, risk factors for thrombosis, values of biomarkers including troponins and natriuretic peptides, echocardiographic results and radiographic results. For many of these elements, additional detail was collected. The malignancy variable was further divided into active cancer (defined as under the care of an oncologist), metastatic malignancy, or inactive cancer, defined as in remission by the patient and no longer under the active care of an oncologist. Patients were followed prospectively for in-hospital adverse outcomes and for survival until 30 days.

The selection of variables, their analysis, model construction and assessment of goodness of fit and validation was performed in accordance with published standards. (Moss M, Wellman DA, Cotsonis GA. An appraisal of multivariable logistic models in the pulmonary and critical care literature. Chest 2003;123:923-928). Twenty-five (25) potential independent variables were identified. The dependent variable was mortality at 30 days. The 25 potential predictor variables were screened for inclusion in the multivariate model using two methods: First, data was analyzed with classification and regression technique (CART, Salford Systems Inc, San Diego, CA). Second, univariate analysis was performed to screen for potentially important differences in means (unpaired t-test) or frequency (Chi square) for the 25 variables between survivors and decedents with variables. Variables with weights >0 in the CART analysis and P<0.1 in univariate analysis were retained. Retained continuous predictor variables (e.g., heart rate) underwent receiver operating characteristic curve analysis to determine the cutoff corresponding to the lowest likelihood ratio negative for prediction of death at 30 days.

Table 1. Results of univariate and CART analysis on 25 candidate predictor variables in the derivation population

| Predictor variable | Survived > 30 days | SD or % | Death within 30 days | SD or % | P value (t-test or Chi-Square) | CART relative weight |
|---|---|---|---|---|---|---|
| Survival status | 357 | 88% | 51 | 13% | NA | NA |
| Age (years) | 62 | 15 | 63 | 13 | 0.716 | 8.6 |
| Systolic blood pressure (mm Hg) | 128 | 25 | 124 | 26 | 0.342 | 7.2 |
| Body weight (pounds) | 180 | 49 | 158 | 40 | 0.004 | 97.8 |
| Heart rate (beats/min) | 97 | 21 | 105 | 22 | 0.009 | 23.9 |
| Respiratory rate (breaths/min) | 21 | 6 | 24 | 8 | 0.004 | 34.1 |
| Sa02 (%) | 95 | 5 | 92 | 7 | 0.000 | 100 |

(continued)

| Predictor variable | Survived > 30 days | SD or % | Death within 30 days | SD or % | P value (t-test or Chi-Square) | CART relative weight |
|---|---|---|---|---|---|---|
| Hemoglobin | 12.0 | 2.4 | 11.4 | 2.4 | 0.119 | 0 |
| Creatinine | 1.1 | 0.7 | 1.0 | 0.8 | 0.937 | 0 |
| White blood count | 11.8 | 34.6 | 11.7 | 6.5 | 0.985 | 0 |
| Male gender | 181 | 51% | 30 | 59% | 0.270 | 0 |
| Respiratory distress | 68 | 19% | 21 | 41% | 0.003 | 64.8 |
| Syncope | 18 | 5% | 2 | 4% | 0.739 | 0 |
| PE most likely diagnosis | 70 | 20% | 6 | 12% | 0.178 | 0 |
| Altered mental status | 14 | 4% | 8 | 16% | <0.001 | 30.3 |
| Do not resuscitate status | 13 | 4% | 10 | 20% | <0.001 | 45.2 |
| Immobilized >72 h | 20 | 6% | 5 | 10% | 0.242 | 0 |
| Hospitalization within previous week | 104 | 29% | 13 | 25% | 0.591 | 0 |
| Prior history of lung disease* | 40 | 11% | 7 | 14% | 0.598 | 0 |
| Prior history of heart failure | 16 | 4% | 3 | 6% | 0.657 | 0 |
| Other comorbidity | 95 | 27% | 11 | 22% | 0.442 | 0 |
| Indwelling venous cathether | 45 | 13% | 8 | 16% | 0.540 | 0 |
| Surgery within previous month | 47 | 13% | 3 | 6% | 0.138 | 0 |
| Prior History of pulmonary embolism | 8 | 2% | 3 | 6% | 0.133 | 0 |
| Unilateral limb swelling | 69 | 19% | 16 | 31% | 0.048 | 7.2 |
| Biomarker or echocardiography abnormal+ | 79 | 34% | 16 | 46% | 0.198 | 0 |

Retained predictors were then entered into a second logistic regression analysis, with the independent predictor being death at 30 days. Model fit was assessed by P values from the Pearson's goodness of fit Chi-Square, Hosmer Lemeshow test, the Pseudo (McFadden) R-square and area under the receiver operating characteristic curve in the derivation dataset (C statistic).

The prognostic accuracy of the model was tested using four prospectively collected datasets of patients diagnosed with PE in the emergency department setting. Studies included one multicenter sample from the US, two single-center samples and one multicenter sample from Europe. The databases were reduced to patients with active cancer and PE, and the percentage mortality estimate (i.e., the predicted probability of death within 30 days) was then determined by taking the antilog and solving the logistic regression equation for the P term. Using a criterion standard of 30 day all-cause mortality, prognostic accuracy was assessed from the area under the curve (AUC) for the receiver operating characteristic curve, with the assumption that lower limit 95% confidence interval for the AUC greater than 0.5 indicated potential validity.

To assess the potential significance of all 25 predictor variables, the original EMPORER database (N=1,880) was reduced to patients with active cancer and PE, excluding patients who had comfort care status only. This process excluded 1,464 patients without active cancer, and 18 patients with active cancer and comfort care only status, leaving 408 patients, of whom 51 (12.5%) died within 30 days of any cause. Table 1 lists the means and frequency data for the 25 predictor variables for survivors compared with patients who died. Table 1 also shows the relative weights of each predictor determined from CART and the univariate P values.

Eight variables had a non-zero weight from CART and a P<0.1 from either the unpaired t-test or Chi-Square and were thus retained for the multivariate model.

The remaining 8 candidate variables were subjected to logistic regression analysis. The lower limit of the 95% confidence interval for the odds ratios associated with each variable is shown in Table 2.

Table 2. Odds ratios for the eight predictor variables from logistic regression

| Variable | | Odds Ratio | Bias corrected 95 % CI |
|---|---|---|---|
| Intercept | | | |
| Patient weight (lb) | | 0.99 | 0.98 to 1 |
| heart rate >99 beats/min | | 2.8 | 1.38 to 5.64 |
| Highest respiratory rate (breaths/min) | | 1.05 | 1.01 to 1.09 |
| SaO2 (%) | | 0.94 | 0.89 to 0.99 |
| Altered mental status | | 4.36 | 1.47 to 13 |
| Respiratory distress | | 2.22 | 1.12 to 4.43 |
| Do not resuscitate | | 4.72 | 1.75 to 12.76 |
| Unilateral limb swelling | | 2.08 | 1 to 4.36 |

The Pearson chi-square goodness of fit P values was 0.94, the Hosmer Lemeshow P value was 0.48, the McFaddens' $R^2$ was 0.21 and the AUC for the receiver operating characteristic curve used on the derivation set was 0.83. Taken together, these data indicate reasonable model fitness predictive accuracy in the derivation set.

The six validation samples contained a total of 14,121 patients, of whom 202 had active cancer and PE+. The database from Europe also did not explicitly record the terms for altered mental status or do not resuscitate but in accordance with original inclusion and exclusion requirements for study enrollment, these were assumed to be absent for all patients. Data were missing [body mass (12), respiratory rate (5) and pulse oximetry (3)] in 20 patients, seven from the European sample and three from the New Zealand sample and these 20 patients were excluded. Thus, 182 patients had complete data, of whom, 27 died within 30 days (mortality 15, 95% CI 10 to 21%). Table 3 presents the mean percentage mortality estimate for patients from each validation sample, stratified by survival status at 30 days.

Table 3. Description of validation samples

| Source | Description* | N** | Outcome, active cancer, PE+ | | Mean estimate of mortality probability |
|---|---|---|---|---|---|
| #1 | Single center, prospective study of PE prognosis (N=200, all PE+) | 19 | Survived > 30 days | 18 | 13% |
| | | | Died within 30 days | 1 | 62% |

(continued)

| Source | Description* | N** | Outcome, active cancer, PE+ | | Mean estimate of mortality probability |
|---|---|---|---|---|---|
| #2 | Single center, prospective study of PE diagnosis (N=178, 24 PE+ ) | 9 | Survived > 30 days | 6 | 6% |
| | | | Died within 30 days | 3 | 39% |
| #3 | Multicenter prospective study of PE diagnosis (N=7,940, 481 PE+) | 70 | Survived > 30 days | 62 | 10% |
| | | | Died within 30 days | 8 | 43% |
| #4 | Multicenter prospective study of PE diagnosis (N=702, 114 PE+ ) | 12 | Survived > 30 days | 10 | 9% |
| | | | Died within 30 days | 2 | 44% |
| #5 | Single center, prospective study of PE diagnosis (N=2,134, 103 PE+ ) | 22 | Survived > 30 days | 14 | 33% |
| | | | Died within 30 days | 8 | 9% |
| #6 | Multicenter prospective study of PE diagnosis (N=3,174, 661 PE+) | 50 | Survived > 30 days | 45 | 16% |
| | | | Died within 30 days | 5 | 45% |
| Total | | 182 | Survived > 30 days | 156 | 12% |
| | | | Died within 30 days | 27 | 44% |
| * PE+ refers to image-proven PE diagnosed at index visit, excludes patients with isolated DVT or PE diagnosed on follow-up, **number with active cancer and PE | | | | | |

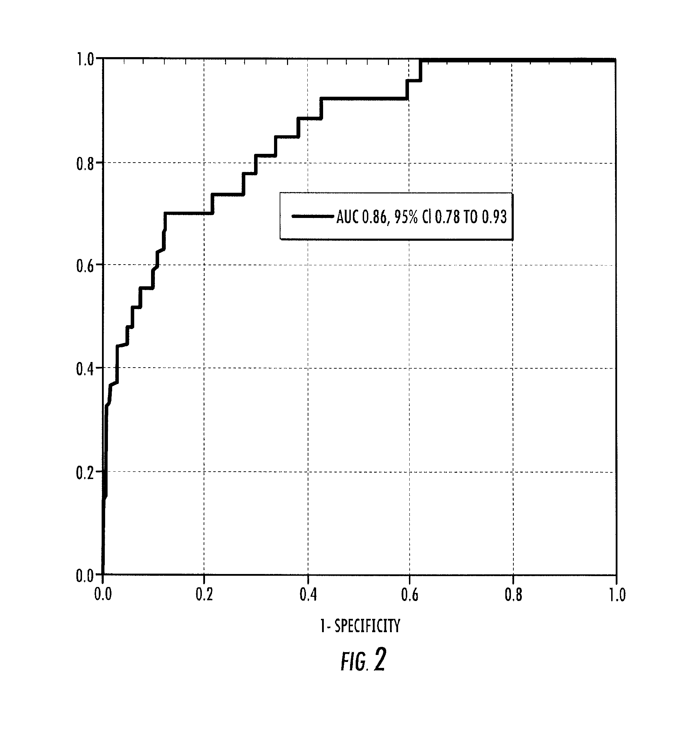

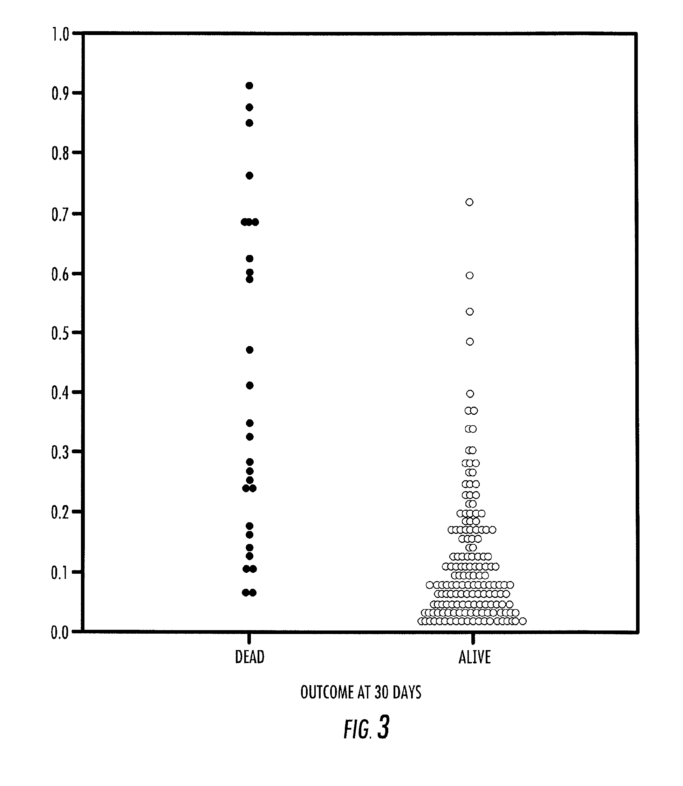

Referring to the figures, Figure 2 shows the receiver operating characteristic curve derived in the aggregated sample of all 182 validation patients. The area under the curve (AUC) was 0.86 (95% CI 0.78 to 0.93) indicating good overall discriminatory value in the validation samples. Figure 3 shows a frequency dot plot of each probability estimate, stratified by survival status at 30 days.

The percentage estimates of mortality were examined for cutoffs that predicted a very low and very high probability of death in the validation sample. The cutoff of 5% produced a very low probability of death; no patient with a mortality estimate of 5% or less died within 30 days (sensitivity 17/17 = 100%, 95% CI 82 to 100%) and 42 patients who survived >30 days had a mortality estimate of 5% or less (specificity 42/132 = 32%, 95% CI 24 to 40%), producing a posterior probability of death equal to 0/42 (95% CI 0 to 8.4%). The cutoff of 50% produced a very high probability of death; 11 patients had a mortality estimate >50%, and 8/11 died (posterior probability 73%, 95% CI 39 to 94%).

In conclusion, the area under the receiver operating characteristic curve was good in the derivation dataset (0.83) and the validation dataset (0.85). Estimates at or below 5% were associated with a very low 30 day mortality rate (0/42, 95% CI 0 to 8.4%).

EXAMPLE

To determine the validity of a range of beta coefficients, 95% confidence intervals were constructed around each coefficient using the bootstrap approach with 1000 interations. (Efron B. The jackknife, the bootstrap and other resampling plans. Philadelphia: Society for Industrial and Applied Mathematics; 1982).

This produced bias-corrected lower limit (LL) and upper limit (UL) values for each beta coefficient as demonstrated in Table 4.

Table 4.

| Parameter | beta coefficient | LL | UL |
|---|---|---|---|
| constant | 3.72 | 3.72 | 3.72 |
| patient body Weight | -0.01 | -0.02 | 0.00 |
| heart rate (hr100) | 1.03 | 0.35 | 1.83 |
| respiratory rate (h rr) | 0.04 | -0.02 | 0.11 |
| Sa02 | -0.06 | -0.13 | 0.00 |
| altered mental state (AMS) | 1.47 | 0.05 | 2.58 |
| respiratory distress | 0.80 | -0.12 | 1.48 |
| "do not resuscitate" | 1.55 | 0.29 | 2.66 |
| (DNR) status | | | |
| unilateral limb swelling (ULS) | 0.73 | -0.16 | 1.53 |

A computer program was then written in Visual Basic to repeatedly draw a random sample between the LL and UL for each beta coefficient to produce a new logistic regression equation characterized by a beta coefficient for each of the eight predictor variables between the LL and UL values. The computer program was constructed with a "For" loop instructing it to repeat this process 1000 times, producing 1000 new equations with each of the eight beta coefficients drawn between the LL and UL values. Each equation was then retested in the derivation database by solving for the value f(z) and then constructing a receiver operating characteristic curve and calculating the area under the curve. For all 1000 equations, the area under the receiver operating characteristic curve was higher than 0.5, indicating a better ability to discriminate which patients in the derivation database would survive for 30 days would be produced by chance alone (e.g., by flipping a coin to predict survival or death).

It will therefore be readily understood by those persons skilled in the art that the present invention is susceptible of broad utility and application. Many embodiments and adaptations of the present invention other than those herein described, as well as many variations, modifications and equivalent arrangements, will be apparent from or reasonably suggested by the present invention and the foregoing description thereof, without departing from the substance or scope of the present invention. Accordingly, while the present invention has been described herein in detail in relation to its preferred embodiment, it is to be understood that this disclosure is only illustrative and exemplary of the present invention and is made merely for purposes of providing a full and enabling disclosure of the invention. The foregoing disclosure is not intended or to be construed to limit the present invention or otherwise to exclude any such other embodiments, adaptations, variations, modifications and equivalent arrangements.

**Claims**

1. A computer-based device comprising:

   computer executable instructions comprising a multivariate, computer-implemented modeling equation to estimate a quantitative short-term prognosis of a patient with active cancer and thrombosis; and
   a computer processor for executing the computer executable instructions.

2. The computer-based device according to claim 1, wherein the thrombosis is pulmonary embolism.

3. The computer-based device according to claim 2, wherein the device estimates a percentage of probability of death from pulmonary embolism within a period of thirty days in the patient with active cancer.

4. The computer-based device according to claim 1, 2 or 3, wherein the multivariate equation is a logistic regression equation.

5. The computer-based device according to any of claims 1 to 4, wherein the multivariate equation comprises predictive factors selected from the group consisting of patient body weight, heart rate, respiratory rate, pulse oximetry, altered mental state, respiratory distress, resuscitation status, and unilateral limb swelling, and a combination thereof.

6. A computer-based device according to claim 3, 4 or 5, wherein the multivariate equation is as set forth in equation (2):

$$P(z) = [1 - [1/(1+e^{z})]] * 100 \qquad\qquad \text{Equation (2)}$$

wherein

$P(z)$ = percentage probability of death within thirty days, and
$z = \beta_0 + \beta_1 x_1 + \beta_2 x_2 + \beta_3 x_3 + \beta_4 x_4 + \beta_5 x_5 + \beta_6 x_6 + \beta_7 x_7 + \beta_8 x_8$
wherein

$\beta_0$ = intercept
$\beta_1$ = patient body weight
$\beta_2$ = heart rate
$\beta_3$ = respiratory rate
$\beta_4$ = pulse oximetry (SaO$_2$)
$\beta_5$ = altered mental state
$\beta_6$ = respiratory distress
$\beta_7$ = "do not resuscitate" status
$\beta_8$ = unilateral limb swelling
$x_1$ = body weight of patient in pounds
$x_2$ = 1 if heart rate of the patient > 99 beats per minute
but $x_2$ = 0 if heart rate of the patient <100 beats per minute
$x_3$ = respiratory rate of the patient in breaths per minute
$x_4$ = pulse oximetry value in % obtained from the patient breathing room air
$x_5$ = 1 if patient has altered mental state but $x_5$ = 0 if patient does not have altered mental state
$x_6$ = 1 if patient has respiratory distress but $x_6$ = 0 if patient does not have respiratory distress
$x_7$ = 1 if patient has "do not resuscitate" status but $x_7$ = 0 if patient does not have "do not resuscitate status"
$x_8$ = 1 if patient has unilateral limb swelling but $x_8$ = 0 but if patient does not have unilateral limb swelling.

7. The computer-based device according to claim 6, wherein

- $\beta_0$ = 3.72, and/or
- $\beta_1$ is in a range of -0.02 to 0, and/or
- $\beta_2$ is in a range of 0.35 to 1.83, and/or
- $\beta_3$ is in a range of -0.02 to 0.11, and/or
- $\beta_4$ is in a range of -0.13 to 0, and/or
- $\beta_5$ is in a range of 0.05 to 2.58, and/or
- $\beta_6$ is in a range of -0.12 to 1.48, and/or
- $\beta_7$ is in a range of 0.29 to 2.66, and/or
- $\beta_8$ is in a range of -0.16 to 1.53.

8. The computer-based device according to any of claims 1 to 7, wherein the device further comprises an output display.

9. A computer-based according to any of claims 3 to 8;
wherein the percentage of 5% or less corresponds to a prognosis of a near zero chance of death within 30 days and/or wherein the percentage of >50% corresponds to a prognosis of a high probability of death within thirty days.

**10.** A method of using a computer-based device to estimate a percentage of probability of death from pulmonary embolism in a patient with active cancer, the method comprising:

estimating a percentage of probability of death from pulmonary embolism in a patient with active cancer based upon a percentage generated by a computer processor of the computer-based device executing computer executable instructions comprising a multivariate, quantitative computer-implemented modeling equation.

**11.** The method of claim 10 further comprising:

entering input data pertaining to a patient with active cancer in the computer-based device having an output display and the computer processor,
processing by the computer processor entered input data according to the multivariate, computer-implemented modeling equation to estimate a percentage of probability of death from pulmonary embolism in the patient with active cancer, and
displaying output data on the output display of the device wherein the output data comprises a percentage probability of death of the patient within a period of thirty days.

**12.** The method according to claim 11, wherein the output data further comprises a recommended course of action for the patient.

**13.** The method according to claim 10, 11 or 12, wherein the multivariate equation is as set forth in equation (2):

$$P(z) = [1 - [1/(1 + e^{z})]] * 100 \qquad\qquad Equation\ (2)$$

wherein

$P(z)$ = percentage probability of death within thirty days, and
$z = \beta_0 + \beta_1 x_1 + \beta_2 x_2 + \beta_3 x_3 + \beta_4 x_4 + \beta_5 x_5 + \beta_6 x_6 + \beta_7 x_7 + \beta_8 x_8$
wherein

$\beta_0$ = intercept
$\beta_1$ = patient body weight
$\beta_2$ = heart rate
$\beta_3$ = respiratory rate
$\beta_4$ = pulse oximetry ($SaO_2$)
$\beta_5$ = altered mental state
$\beta_6$ = respiratory distress
$\beta_7$ = "do not resuscitate" status
$\beta_8$ = unilateral limb swelling
$x_1$ = body weight of patient in pounds
$x_2$ = 1 if heart rate of the patient > 99 beats per minute
but $x_2$ = 0 if heart rate of the patient <100 beats per minute
$x_3$ = respiratory rate of the patient in breaths per minute
$x_4$ = pulse oximetry value in % obtained from the patient breathing room air
$x_5$ = 1 if patient has altered mental state but $x_5$ = 0 if patient does not have altered mental state
$x_6$ = 1 if patient has respiratory distress but $x_6$ = 0 if patient does not have respiratory distress
$x_7$ = 1 if patient has "do not resuscitate" status but $x_7$ = 0 if patient does not have "do not resuscitate status"
$x_8$ = 1 if patient has unilateral limb swelling but $x_8$ = 0 but if patient does not have unilateral limb swelling.

**14.** The method according to claim 13, wherein

- $\beta_0$ = 3.72, and/or
- $\beta_1$ is in a range of -0.02 to 0, and/or
- $\beta_2$ is in a range of 0.35 to 1.83, and/or
- $\beta_3$ is in a range of -0.02 to 0.11, and/or
- $\beta_4$ is in a range of -0.13 to 0, and/or

- $\beta_5$ is in a range of 0.05 to 2.58, and/or
- $\beta_6$ is in a range of -0.12 to 1.48, and/or
- $\beta_7$ is in a range of 0.29 to 2.66, and/or
- $\beta_8$ is in a range of -0.16 to 1.53.

15. The method according to any of claims 10 to 14, wherein the percentage of 5% or less corresponds to a prognosis of a near zero chance of death within 30 days and/or wherein the percentage of >50% corresponds to a prognosis of a high probability of death within thirty days.

## INPUT DATA

| | |
|---|---|
| BODY WEIGHT IN POUNDS | 170 |
| HEART RATE | 105 |
| RESPIRATORY RATE | 22 |
| PULSE OXIMETRY READING | 98 |
| RESPIRATORY DISTRESS? (1 IF YES, 0 IF NO) | 0 |
| ALTERED MENTAL STATUS? (1 IF YES, 0 IF NO) | 0 |
| DO NOT RESUSCITATE STATUS? (1 IF YES, 0 IF NO) | 0 |
| UNILATERAL LIMB SWELLING? (1 IF YES, 0 IF NO) | 0 |

## OUTPUT

PROBABILITY OF DEATH WITHIN 30 DAYS ⟹ 8%

TREATMENT RECOMMENDATION ⟹ ADMIT FOR ANTICOAGULATION

*FIG. 1*

*FIG. 2*

OUTCOME AT 30 DAYS

FIG. 3

Europäisches
Patentamt

European
Patent Office

Office européen
des brevets

## EUROPEAN SEARCH REPORT

**Application Number**

EP 12 18 3425

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | D. AUJESKY ET AL: "Derivation and Validation of a Prognostic Model for Pulmonary Embolism", AMERICAN JOURNAL OF RESPIRATORY AND CRITICAL CARE MEDICINE, vol. 172, no. 8, 1 October 2005 (2005-10-01), pages 1041-1046, XP055048936, ISSN: 1073-449X, DOI: 10.1164/rccm.200506-8620C * the whole document * | 1-15 | INV. G06F19/00 |
| X | A. A. KHORANA ET AL: "Development and validation of a predictive model for chemotherapy-associated thrombosis", BLOOD, vol. 111, no. 10, 15 May 2008 (2008-05-15), pages 4902-4907, XP055048938, ISSN: 0006-4971, DOI: 10.1182/blood-2007-10-116327 * the whole document * | 1-15 | |
| X | WO 2006/113987 A1 (CADUCEUS INFORMATION SYSTEMS I [CA]; DRANITSARIS GEORGE [CA]; VINCENT) 2 November 2006 (2006-11-02) * page 4, line 10 - line 24 * * page 7, line 9 - line 26 * * page 11, line 26 - line 31 * * page 15, line 17 - line 21 * * page 16, line 30 - page 17, line 11 * * page 24, line 19 - page 25, line 13 * * page 33, line 20 - page 34, line 11 * * page 37, line 13 - line 26 * * page 38, line 28 - page 39, line 6 * * claims 1-5 * | 1-15 | TECHNICAL FIELDS SEARCHED (IPC) G06F |

The present search report has been drawn up for all claims

| Place of search | Date of completion of the search | Examiner |
|---|---|---|
| Munich | 9 January 2013 | Sisk, Aisling |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 18 3425

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2013

| Patent document cited in search report | Publication date | Patent family member(s) | | Publication date |
|---|---|---|---|---|
| WO 2006113987 A1 | 02-11-2006 | CA | 2650562 A1 | 02-11-2006 |
| | | US | 2010204920 A1 | 12-08-2010 |
| | | WO | 2006113987 A1 | 02-11-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61532349 B

**Non-patent literature cited in the description**

- **MOSS M ; WELLMAN DA ; COTSONIS GA.** An appraisal of multivariable logistic models in the pulmonary and critical care literature. *Chest,* 2003, vol. 123, 923-928